# EUROPEAN PATENT SPECIFICATION

(11) **EP 2 000 088 B1**
(45) Date of publication and mention of the grant of the patent: **22.09.2010**
(21) Application number: 08251922.4
(22) Date of filing: 03.06.2008
(51) Int. Cl.: A61B 5/06

(54) **Intracorporeal location system with movement compensation**
Intrakorporales Positionssystem mit Bewegungsausgleich
Système à localisation intracorporelle doté d'une compensation de mouvement

(30) Priority: 04.06.2007 US 941767 P; 29.05.2008 US 129012
(43) Date of publication of application: 10.12.2008
(73) Proprietor: Biosense Webster, Inc., Diamond Bar, CA 91765 (US)
(72) Inventor: Govari, Assaf, 34400 Haifa (IL); Altmann, Andres Claudio, 34614 Haifa (IL); Levin, Alexander, 35590 Haifa (IL)
(74) Representative: Small, Gary James

(56) References cited:
- EP-A- 0 974 936
- WO-A-02/081022
- US-A1- 2002 165 448

## Description

### FIELD OF THE INVENTION

The present invention relates generally to medical instruments, and specifically to position sensing systems for tracking the location of invasive devices inside the body.

### BACKGROUND OF THE INVENTION

In intracardiac tracking systems, such as CARTO^{™} (produced by Biosense Webster, Diamond Bar, California), the position coordinates of a catheter inside the heart are determined relative to a reference location outside the patient's body. In CARTO, for example, both the catheter and a reference pad under the patient's back contain miniature coils, which sense the amplitude and direction of a magnetic field. As the patient breathes, however, the resulting movement of the patient's thorax causes the heart to shift position relative to the reference pad, so that the coordinates of the catheter will change during the respiratory cycle even while the catheter is stationary relative to the heart.

U.S. Patent 5,391,199 describes an apparatus and method for mapping and treatment of cardiac arrhythmias using a catheter with location sensing capability inside the heart. To correct for displacement of the heart chamber that may occur because of breathing or patient movement, a set of more than two locatable catheters may be placed at specific points in the heart chamber during the mapping procedures to serve as reference catheters. The location of these reference catheters supplies the necessary information for proper three-dimensional correspondence of the mapping catheter location within the heart chamber.

### SUMMARY OF THE INVENTION

The use of a reference probe, such as a catheter, in a stable position inside the heart can enhance accuracy in measuring the position of an active device, such as a mapping catheter, as the active device is maneuvered within the heart. The reference probe is held stationary and serves as a reference point for measuring the relative coordinates of the active device. Since respiratory motion affects both the reference catheter and the active device in roughly the same way, the effect of respiratory motion on the coordinates of the active device can thus be largely eliminated.

In practice, however, it can be difficult to maintain the stability of the reference probe. Even small displacements of the reference probe can seriously compromise the accuracy of measurement of the position of the active device. Embodiments of the present invention that are described hereinbelow provide methods and systems that can be used to address this problem, and thus provide accurate position readings even when the reference probe is not entirely stable.

There is provided, in accordance with an embodiment of the present invention, an apparatus for position tracking, including:
an internal reference probe, which includes a first position transducer and is configured to be placed in a reference location within a heart of a subject;
an active device, which includes a second position transducer and is configured to be introduced into the heart; and
a positioning processor, which is coupled to collect and process first location coordinates of the internal reference probe in a fixed frame of reference, using the first position transducer, during one or more respiratory cycles of the subject so as to define a range of the location coordinates corresponding to the reference location, and to collect second location coordinates of the active device in the fixed frame of reference, using the second position transducer, and to jointly process the first and second location coordinates so as to find relative location coordinates of the active device in a cardiac frame of reference,
wherein the positioning processor is configured, after defining the range of the location coordinates corresponding to the reference location, to detect a deviation of the first location coordinates from the range, thereby identifying a displacement of the reference probe from the reference location, and to correct the relative location coordinates so as to compensate for the displacement.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a cardiac catheterization system, in accordance with an embodiment of the present invention;
Fig. 2 is a schematic side view of the distal end of a catheter, in accordance with an embodiment of the present invention;
Figs. 3 and 4 are vector diagrams that schematically illustrate a method for finding location coordinates of a catheter, in accordance with an embodiment of the present invention;
Fig. 5 is a flow chart that schematically illustrates a method for finding location coordinates of a catheter, in accordance with an embodiment of the present invention; and
Fig. 6 is a graphical representation of sets of position measurements of a reference catheter, in accordance with an embodiment of the present invention.

### DETAILED DESCRIPTION OF EMBODIMENTS

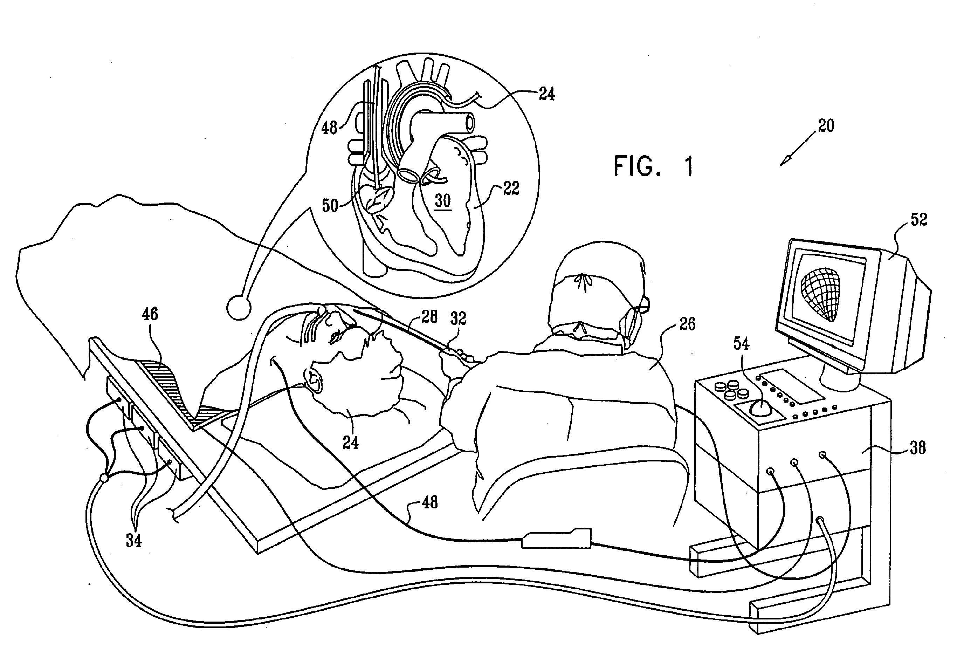

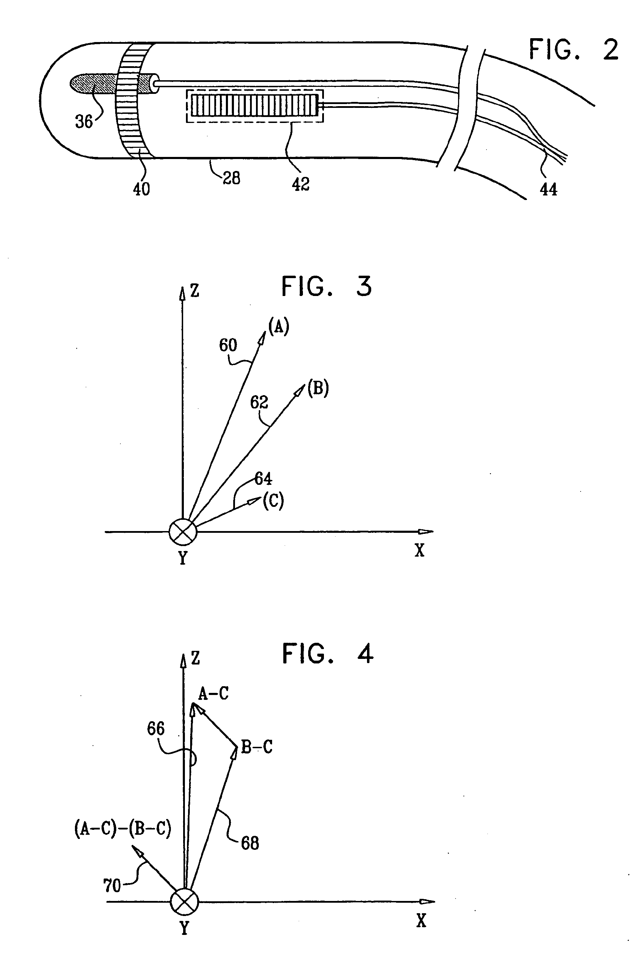

Reference is now made to Figs. 1 and 2, which schematically illustrate a system 20 for catheterization of a heart 22 of a patient 24, in accordance with an embodiment of the present invention. The system comprises, inter alia, a catheter 28, which is inserted by a physician 26 into a chamber 30 of the heart through a vein or artery. Fig. 1 is a pictorial view of the system as a whole, while Fig. 2 shows details of the distal end of the catheter.

Catheter 28 may serve as an active device for a variety of purposes, including diagnostic applications such as mapping or imaging of the heart, as well therapeutic applications, such as ablation-based treatment of arrhythmias. Catheter 28 typically comprises a handle 32 for operation of the catheter by the physician. Suitable controls (not shown) on the handle enable the physician to steer, position and orient the distal end of the catheter as desired. In the example configuration shown in Fig. 2, catheter 24 comprises, at its distal end, a number of functional components, including an electrode 40, which may be used for electrical sensing and/or ablation inside heart 22, as well as an acoustic transducer 42, which may be used for ultrasonic imaging. These components, however, are shown solely by way of illustration, and the principles of the present invention are equally applicable to other types of catheters, as well as other invasive devices.

System 20 comprises a positioning sub-system, which measures location and orientation coordinates of catheter 28. (Throughout this patent application and in the claims, the term "location" refers to the spatial coordinates of the catheter, and the term "orientation" refers to its angular coordinates. The term "position" refers to the full positional information of the catheter, which may comprise both location and orientation coordinates.) For the purpose of these coordinate measurements, the distal end of catheter 28 contains a position sensor 36, which generates signals that are used by a positioning processor 38 in computing position coordinates of the catheter inside the heart. Sensor 36, as well as the functional components in catheter 28, are connected to processor 38 by cables 44 running through the catheter.

In one embodiment, the positioning sub-system comprises a magnetic position tracking system that determines the location and orientation of catheter 28. The positioning sub-system generates magnetic fields in a predefined working volume the vicinity of heart 22 and senses these fields at the catheter. For this purpose, the positioning sub-system typically comprises a set of external radiators, such as field generator coils 34, which are located in fixed, known positions external to patient 24 and generate electromagnetic fields in the vicinity of heart 22. The fields generated by coils 34 thus define a fixed frame of reference. Position sensor 36 in this embodiment may comprise one or more coils, which sense the fields generated by coils 34 and convey to processor 38 signals that are proportional to directional components of the fields. The processor typically receives, amplifies, filters, digitizes, and processes these signals in order to determine the coordinates of the sensor in the frame of reference of coils 34. In an alternative embodiment, a radiator, such as a coil, in the catheter generates electromagnetic fields, which are received by sensors outside the patient's body.

The principles of operation of this sort of positioning sub-system are further described in the above-mentioned U.S. Patent 5,391,199. Other position tracking systems that operate in this general manner are described, for example, in U.S. Patents 6,690,963, 6,618,612 and 6,332,089, and U.S. Patent Application Publications 2002/0065455 A1, 2004/0147920 A1 and 2004/0068178 A1. Integration of the positioning sub-system with the ultrasonic imaging capabilities of transducer 42 is described in U.S. Patent Application Publication 2006/0241445. Although the positioning sub-system of Fig. 1 uses magnetic fields, the methods described below may likewise be implemented using any other suitable positioning sub-system, such as systems based on electrical impedance or acoustical measurements. The term "position transducer," in the context of the present patent application and in the claims, thus refers generically to any sort of component that can be used in an invasive device, such as a catheter, to generate signals indicative of the coordinates of the component, whether by transmission or reception of radiation. Magnetic position sensor 36 is one type of position transducer and is described here solely by way of illustration, and not limitation.

Position sensor 36 is located within the distal end of catheter 28, adjacent to electrode 40 and transducer 42, as shown in Fig. 2. Typically, the mutual locational and orientational offsets between the position sensor, electrode, and transducers are constant. These offsets are used by positioning processor 38 to derive the coordinates of electrode 40 and transducer 42, given the measured position of position sensor 36.

In order to reduce possible errors in the position coordinates of catheter 28 that are computed by processor 38, system 20 comprises two position reference elements:
- A reference pad 46, which is typically attached to the back of patient 24. Pad 46 comprises one or more position transducers, such as a position sensor similar to sensor 36 in catheter 28. (In fact, the pad may itself simply comprise another catheter like catheter 28.) The signal that is output by the sensor in pad 46 thus provides a stable position reference, which does not move during the procedure carried out by physician 26 unless the patient himself moves. The reference pad may, for example, be a QWIKSTAR back pad, which is supplied as part of the above-mentioned CARTO system.
- A reference catheter 48, which is typically inserted by physician 26 into heart 22 and is positioned within the heart at a known, stable reference location. Catheter 48 likewise comprises one or more position transducers and thus serves as the reference probe for finding relative location coordinates of catheter 28 in a cardiac frame of reference, i.e., a reference frame that is anchored in heart 22, rather than external to the patient's body, as described further hereinbelow. The reference catheter may, for example, be a CARTO NAVISTAR catheter.

In the inset in Fig. 1, for example, catheter 48 is fed through the superior vena cava into the right atrium of heart 22, and its distal end is inserted into a coronary sinus 50. In general, in this sort of position, catheter 48 is not expected to move relative to the heart during the procedure (and the coronary sinus itself moves relatively little in the course of the heart cycle). On the other hand, respiratory motion of the thorax of patient 24 will cause the position of catheter 48, relative to reference pad 46, to shift cyclically along with catheter 28 and the rest of the patient's heart.

Processor 38 jointly processes the coordinates of sensor 36 with the coordinates of catheter 48 in order to find relative location coordinates of catheter 28 that neutralize the effects of respiration and other patient movement, as is described further hereinbelow. The coordinates of reference pad 46 may also be used in this computation in order to enhance the stability of the coordinates against patient movement and changes in the operating environment. The processor typically uses the resulting accurate position measurements in generating maps and/or images of the heart, which are presented on a display 52, and in accurately tracking the location of catheter 28 during diagnostic and therapeutic procedures. A user, such as physician 26, may interact with the display and may control processor using an input device 54, such as a pointing device and/or a keyboard.

Typically, positioning processor 38 comprises a general-purpose computer processor, which is programmed in software to carry out the functions described herein. The software may be downloaded to the computer in electronic form, over a network, for example, or it may, alternatively or additionally, be stored on tangible media, such as optical, magnetic or electronic memory media. The functions of the positioning processor may be implemented using a dedicated computer, or they may be integrated with other computing functions of system 20. Additionally or alternatively, at least some of the processing functions may be performed using dedicated hardware.

Fig. 3 is a vector diagram that schematically illustrates location coordinates computed by processor 38, in accordance with an embodiment of the present invention. Although the diagram is two-dimensional (for the sake of simplicity and visual clarity), in practice the processor determines location coordinates in system 20 in three dimensions. Furthermore, although the embodiments described hereinbelow relate specifically to correction of location coordinates, the principles of the present invention may similarly be applied, mutatis mutandis, in reducing errors that may occur in the orientation coordinates of catheter 28, as well.

Fig. 3 shows the following vectors:
- A vector 60 (marked A) represents the coordinates of sensor 36 in catheter 28, which are computed by processor 38 relative to the fixed, external frame of reference of field generator coils 34. These are absolute coordinates, which do not take into account any movement of patient 24, which may occur due to respiration or any other cause.
- Another vector 62 (marked B) represents the coordinates of reference catheter 48, which is supposed to remain stationary (relative to heart 22) within coronary sinus 50. These are, again, absolute coordinates. They are expected to shift cyclically due to respiration of patient 24 and may also exhibit a fixed shift if either patient 24 moves during the procedure or catheter 48 moves within heart 22. Methods for dealing with these sorts of shifts are described hereinbelow.
- A vector 64 (marked C) represents the coordinates of reference pad 46, which are not expected to change at all.

Vectors 60 and 62 are also expected to shift cyclically due to the beating motion of heart 22. In order to neutralize this motion component, the coordinate measurements may be synchronized with the heart cycle, by gating signal capture relative to a body-surface electrocardiogram (ECG) signal or a local intracardiac electrogram. The intracardiac electrogram may be detected, for example, by an electrode on reference catheter 48. A certain point in the heart cycle, such as the peak of the QRS wave in the ECG or a peak in the electrogram, is chosen as an annotation point, and the measurements of vectors 60 and 62 are made at the annotation point in each heart cycle or at a certain fixed delay relative to the annotation point. Once the effect of the motion of the heart itself has been neutralized in this fashion, vectors 60 and 62 are expected to change identically in response to patient motion (including respiratory motion and small shifts of the patient's body).

Fig. 4 is a vector diagram that schematically illustrates a method applied by processor 38 in computing coordinates of catheter 28 relative to heart 22, in accordance with an embodiment of the present invention. As shown in this figure, vectors 60 and 62 (A and B in Fig. 3) are referred to vector 64 (C) to give location vectors 66 (A-C) and 68 (B-C), corresponding to the location coordinates of catheters 28 and 48, respectively, in the frame of reference of pad 46. This frame of reference is expected to be stationary, except to the extent that patient 24 moves during the procedure. When the patient does move, this movement is neutralized by referencing of the coordinates to pad 46.

Processor 38 subtracts vector 68 from vector 66 to give a relative location vector 70 of sensor 36 in the cardiac frame of reference defined by catheter 48. The resulting vector 70 is given by (A-C) - (B-C) = (A-B). As illustrated by this formula, vector 64 drops out of the final calculation, so that pad 46 is not critical in finding the relative location coordinates of catheter 28. The additional reference provided by pad 46 is useful, however, in detecting and compensating for displacement of catheter 48 from its reference location, as will be explained further hereinbelow.

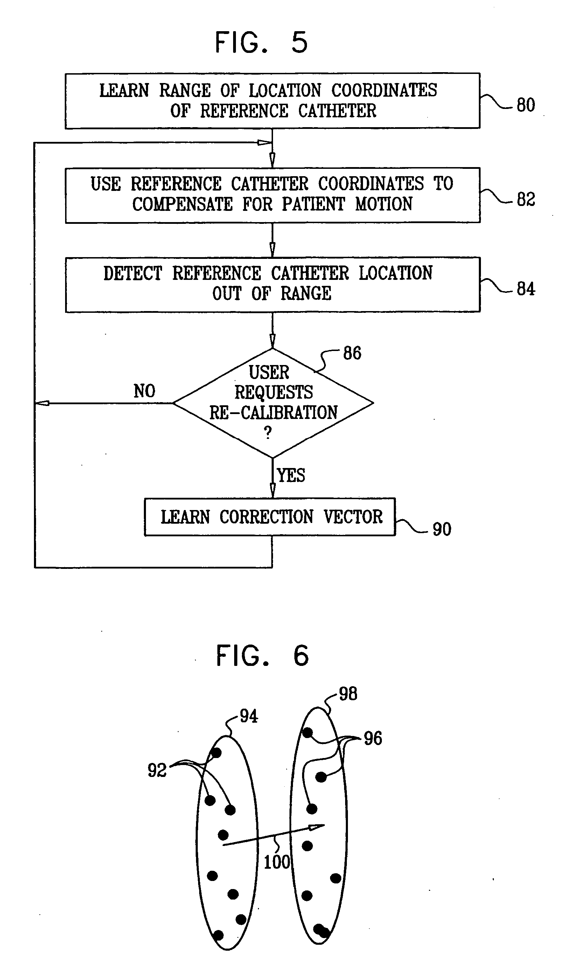

Fig. 5 is a flow chart that schematically illustrates a method for finding location coordinates of catheter 28 in system 20. The method assumes, as its starting point, that reference catheter 48 has been inserted into heart 22 and placed in coronary sinus 50, as shown in Fig. 1. Processor 38 collects and processes coordinate readings provided by the position sensor in reference catheter 48 over a number of respiratory cycles of patient 24, at a location learning step 80. System 20 may alert physician 26 that the learning phase is in progress, so that the physician can make sure not to do anything that might move the reference catheter accidentally during this phase.

The coordinate readings collected during step 80 are typically referred to the measured coordinates of reference pad 46, as explained above with reference to Fig. 4. Alternatively, if patient movement (other than respiratory motion) can be neglected, the "raw" coordinates of catheter 48 in the external frame of reference of field generator coils 34 may be used. As noted above, the readings are typically taken at a certain reference point in the patient's heart cycle. Processor 38 applies statistical processing to the coordinate readings in order to define the range of locations that is normally traversed by catheter 48 over the course of a respiration cycle.

Once the learning phase is complete, physician 26 may begin to move catheter 28 within heart 22 in order to perform a diagnostic or therapeutic procedure. Processor 38 receives signals from sensor 36 in catheter 28, as well as from the position sensors in catheter 48 and pad 46. The processor processes these signals to find the raw coordinates of catheters 28 and 48 and of pad 46 in the external frame of reference, and then jointly processes these raw coordinates to find the relative coordinates of catheter 28 in the cardiac frame of reference, as explained above with reference to Figs. 3 and 4. In this way, the processor compensates for the effect of patient respiration (as well as other possible movement of the patient) on the location coordinates of catheter 28, at a motion compensation step 82. In other words, the position of catheter 28 that is presented to physician 26 (in a map on display 52, for example) reflects the actual position of the catheter in the frame of reference of the heart, irrespective of the overall movement of the heart due to respiratory motion or other causes.

Processor 38 continually monitors the coordinates of reference catheter 48 to ensure that they remain within the range that was learned at step 80. If the processor determines that the coordinates have deviated from the range by more than a permitted threshold, it alerts physician 26, at a reference movement detection step 84. For example, the processor may raise an alert when the reference catheter coordinates are more than 2 mm outside the range that was learned previously. In performing this measurement, it is desirable that processor 38 refer the coordinates of catheter 48 to pad 46, in order to distinguish actual displacement of the reference catheter in the heart from changes in the raw coordinates of the reference catheter that may occur due to movement of the patient during the procedure.

Typically, after receiving the alert, physician 26 has the choice of instructing processor 38 to correct and compensate for the displacement of reference catheter 48, or simply to continue with the procedure, at a user input step 86. Alternatively, system 20 may decide autonomously to perform the correction. In either case, when the decision has been made to correct the coordinates, processor 38 learns the new range of location coordinates of the reference catheter, at a correction step 90. This step is similar to step 80. The processor compares the new range to the previous range in order to compute a correction vector, which estimates the displacement of the reference catheter.

Once the processor has found the correction vector, system 20 returns to normal operation at step 82. The processor now subtracts out the correction vector in computing the relative coordinates of catheter 28, and thus compensates for the displacement of reference catheter 48. As a result, system 20 will continue to display the relative position of catheter 28 in heart 22 as though the reference catheter had not moved from its original position. If the reference catheter moves again subsequently, the processor will repeat steps 84-90, and the new correction vector will then be added cumulatively to the previous correction vector.

Fig. 6 is a schematic, graphical representation of sets of position measurements 92, 96 of reference catheter 48, illustrating the operation of system 20 at steps 80 and 90 in accordance with an embodiment of the present invention. Processor 38 gathers measurement points 92 at step 80. As noted earlier, the measurement points are collected over the course of one or more respiratory cycles, typically at the same annotation point in the patient's heart cycle. Assuming the patient to be supine, respiration causes mainly vertical motion of the reference catheter, as illustrated by points 92. The locations of points 92 defines a range 94. In this case, the range is the smallest ellipse with a vertical major axis that contains all of points 92, but other methods may alternatively be used to define the range.

Although the range is shown in Fig. 6 as comprising simply a "cloud" of coordinates, the range may, additionally or alternatively, be defined by kinematic features, such as the path and/or speed of movement from point to point. In this case, displacement of the reference catheter may be detected at step 90 not only based on excursion of the coordinates outside the "cloud," but also based on kinematic deviation.

In any case, after the processor has detected movement of the reference catheter outside the expected range at step 84, it gathers a new set of measurement points 96 at step 90. Typically, this procedure can be completed over one or a few respiratory cycles. Points 96 define a new range 98. The processor computes a correction vector 100 by comparing ranges 94 and 98. For example, as shown in Fig. 6, the correction vector may be given by the vector displacement between the centers of mass of old range 94 and new range 98.

If reference catheter 48 includes one or more electrodes, displacement of the reference catheter may also be detected electrically. For example, the timing of a peak in the local electrogram detected by the reference catheter electrode may be compared with the QRS peak in the body-surface ECG. A shift in this timing may indicate that the reference catheter has moved. As another example, if the reference catheter has multiple electrodes at different positions along its length, a relative shift in the locations of the peaks in the electrograms detected by the different electrodes may likewise indicate that the reference catheter has moved. These timing changes are independent of the actual position measurements and are generally not sensitive to patient motion.

It will be appreciated that the embodiments described above are cited by way of example, and that the present invention is not limited to what has been particularly shown and described hereinabove. Rather, the scope of the present invention is defined in the appended claims.

## Claims

1. Apparatus for position tracking, comprising:
an internal reference probe (48), which comprises a first position transducer and is configured to be placed in a reference location within a heart (22) of a subject (24);
an active device (28), which comprises a second position transducer (36) and is configured to be introduced into the heart; and
a positioning processor (38), which is coupled to collect and process first location coordinates of the internal reference probe (48) in a fixed frame of reference, using the first position transducer, during one or more respiratory cycles of the subject so as to define a range of the location coordinates corresponding to the reference location, and to collect second location coordinates of the active device (28) in the fixed frame of reference, using the second position transducer (36), and to jointly process the first and second location coordinates so as to find relative location coordinates of the active device (28) in a cardiac frame of reference,
wherein the positioning processor is configured, after defining the range of the location coordinates corresponding to the reference location, to detect a deviation of the first location coordinates from the range, thereby identifying a displacement of the reference probe (48) from the reference location, and to correct the relative location coordinates so as to compensate for the displacement.

2. The apparatus according to claim 1, wherein the internal reference probe and the active device comprise catheters.

3. The apparatus according to claim 1, and comprising magnetic field generators (34), which are configured to generate magnetic fields that define the fixed frame of reference, wherein the first and second position transducers comprise magnetic field sensors, which are configured to output position signals responsively to magnetic fields.

4. The apparatus according to claim 1, wherein the positioning processor (38) is configured to take a vector difference between the first and second location coordinates in order to find the relative location coordinates.

5. The apparatus according to claim 1, and comprising a reference pad (46) that is fixed to a body of the subject, wherein the positioning processor (38) is coupled to collect third location coordinates, in the fixed frame of reference, of the reference pad and to refer at least the first location coordinates to the third location coordinates in order to find the relative location coordinates.

6. The apparatus according to claim 1, wherein the positioning processor (38) is configured to compare the first location coordinates to the third location coordinates so as to determine whether the deviation is due to the displacement of the reference probe (48) from the reference location or due to a movement of the body of the subject (24).

7. The apparatus according to claim 1, wherein the positioning processor (38) is configured to compute a correction vector responsively to the displacement, and to apply the correction vector in finding the relative location coordinates based on the first and second location coordinates.

8. The apparatus according to claim 7, wherein the positioning processor (38) is configured to collect and process further location coordinates of the internal reference probe (48), after detecting the deviation, so as to define a new range of the location coordinates corresponding to the reference location, and to compute the correction vector by comparing the new range to the range that was defined by collecting and processing the first location coordinates.

9. The apparatus according to claim 1, wherein the internal reference probe (48) comprises an electrode, and wherein the positioning processor (38) is coupled to receive a local electrogram signal from the electrode at the reference location within the heart (22) and to detect the deviation by detecting a change in the local electrogram signal.

## Patentansprüche

1. Vorrichtung für die Positionsverfolgung, die aufweist:
eine interne Referenzsonde (48), die einen ersten Positionstransducer aufweist und so ausgelegt ist, dass sie an einen Referenzort innerhalb eines Herzens (22) einer Person (24) gebracht werden kann;
eine aktive Einheit (28), die einen zweiten Positionstransducer ((36) aufweist und so ausgelegt ist, dass sie in das Herz eingeführt werden kann; und
einen Positionierprozessor (38), der so gekoppelt ist, dass er erste Ortskoordinaten der internen Referenzsonde (48) in einem festen Referenzrahmen sammelt und verarbeitet, wobei der erste Positionstransducer verwendet wird, während einem oder mehrerer Atmungszyklen der Person, um so einen Bereich der Ortskoordinaten zu definieren, der dem Referenzort entspricht, und dass er zweite Ortskoordinaten der aktiven Einheit (28) in dem festen Referenzrahmen sammelt, wobei der zweite Positionstransducer (36) verwendet wird, und dass er die ersten und zweiten Ortskoordinaten gemeinsam verarbeitet, um so relative Ortskoordinaten der aktiven Einheit (28) in einem herzbezogenen Referenzrahmen zu finden,
wobei der Positionierprozessor so ausgelegt ist, dass er nach dem Definieren des Bereiches der Ortskoordinaten, der dem Referenzort entspricht, eine Abweichung der ersten Ortskoordinaten von dem Bereich erfasst, so dass eine Verlagerung der Referenzsonde (48) aus dem Referenzort identifiziert wird, und dass er die relativen Ortskoordinaten korrigiert, um so die Verlagerung zu kompensieren.

2. Vorrichtung nach Anspruch 1, bei der die interne Referenzsonde und die aktive Einheit Katheter aufweisen.

3. Vorrichtung nach Anspruch 1 und mit Magnetfeldgeneratoren (34), die so ausgelegt sind, dass sie Magnetfelder erzeugen, welche den festen Referenzrahmen definieren, wobei der erste und der zweite Positionstransducer Magnetfeldsensoren aufweisen, die so ausgelegt sind, dass sie Positionssignale ansprechend auf Magnetfelder ausgeben.

4. Vorrichtung nach Anspruch 1, bei der der Positionierprozessor (38) so ausgelegt ist, dass er eine Vektordifferenz zwischen den ersten und den zweiten Ortskoordinaten nimmt, um die relativen Ortskoordinaten aufzufinden.

5. Vorrichtung nach Anspruch 1 und mit einer Referenzanschlussfläche (46), die an einem Körper der Person festgelegt ist, wobei der Positionierprozessor (38) so gekoppelt ist, dass er in dem festen Referenzrahmen der Referenzanschlussfläche dritte Ortskoordinaten sammelt und dass er wenigstens die ersten Ortskoordinaten zu den dritten Ortskoordinaten in Bezug setzt, um die relativen Ortskoordinaten aufzufinden.

6. Vorrichtung nach Anspruch 1, bei der der Positionierprozessor (38) so ausgelegt ist, dass er die ersten Ortskoordinaten mit den dritten Ortskoordinaten vergleicht, um so festzustellen, ob die Abweichung von der Verlagerung der Referenzsonde (48) aus dem Referenzort oder von einer Bewegung des Körpers der Person (24) verursacht worden ist.

7. Vorrichtung nach Anspruch 1 bei der der Positionierprozessor (38) so ausgelegt ist, dass er einen Korrekturvektor entsprechend der Verlagerung berechnet und dass er den Korrekturvektor beim Auffinden der relativen Ortskoordinaten basierend auf den ersten und den zweiten Ortskoordinaten anwendet.

8. Vorrichtung nach Anspruch 7, bei der der Positionierprozessor (38) so ausgelegt ist, dass er nach dem Erfassen der Abweichung weitere Ortskoordinaten der internen Referenzsonde (48) sammelt und verarbeitet, um so einen neuen Bereich für die Ortskoordinaten zu definieren, der dem Referenzort entspricht, und dass er den Korrekturvektor berechnet, indem er den neuen Bereich mit dem Bereich vergleicht, der durch Sammeln und Verarbeiten der ersten Ortskoordinaten definiert worden ist.

9. Vorrichtung nach Anspruch 1, bei der die interne Referenzsonde (28) eine Elektrode aufweist und bei der der Positionierprozessor (38) so gekoppelt ist, dass er ein lokales Elektrogramm-Signal von der Elektrode an dem Referenzort innerhalb des Herzens (22) erhält und die Abweichung durch Erfassen einer Änderung in dem lokalen Elektrogramm-Signal bestimmt.

## Revendications

1. Dispositif de suivi de position, comprenant :
une sonde de référence interne (48), qui comprend un premier transducteur de position et qui est configurée pour être placée à un emplacement de référence dans un coeur (22) d'un sujet (24) ;
un dispositif actif (28), qui comprend un deuxième transducteur de position (36) et qui est configuré pour être introduit dans le coeur ; et
un processeur de positionnement (38), qui est couplé pour collecter et traiter des premières coordonnées d'emplacement de la sonde de référence interne (48) dans un système de référence fixé, en utilisant le premier transducteur de position, pendant un ou plusieurs cycles respiratoires du sujet de manière à définir une plage des coordonnées d'emplacement correspondant à l'emplacement de référence, et pour collecter des deuxièmes coordonnées d'emplacement du dispositif actif (28) dans le système de référence fixé, en utilisant le deuxième transducteur de position (36), et pour traiter conjointement les premières et deuxièmes coordonnées d'emplacement de manière à trouver des coordonnées d'emplacement relatives du dispositif actif (28) dans un système cardiaque de référence,
dans lequel le processeur de positionnement est configuré pour, après la définition de la plage des coordonnées d'emplacement correspondant à l'emplacement de référence, détecter un écart des premières coordonnées d'emplacement par rapport à la plage, identifiant de ce fait un déplacement de la sonde de référence (48) par rapport à l'emplacement de référence, et pour corriger les coordonnées d'emplacement relatives de manière à compenser le déplacement.

2. Dispositif selon la revendication 1, dans lequel la sonde de référence interne et le dispositif actif comprennent des cathéters.

3. Dispositif selon la revendication 1, et comprenant des générateurs de champ magnétique (34), qui sont configurés pour générer des champs magnétiques qui définissent le système de référence fixé, dans lequel les premier et deuxième transducteurs de position comprennent des capteurs de champ magnétique, qui sont configurés pour délivrer des signaux de position en réponse aux champs magnétiques.

4. Dispositif selon la revendication 1, dans lequel le processeur de positionnement (38) est configuré pour prendre une différence vectorielle entre les premières et deuxièmes coordonnées d'emplacement afin de trouver les coordonnées d'emplacement relatives.

5. Dispositif selon la revendication 1, et comprenant un tapis de référence (46) qui est fixé à un corps du sujet, dans lequel le processeur de positionnement (38) est couplé pour collecter des troisièmes coordonnées d'emplacement, dans le système de référence fixé, du tapis de référence et pour référencer au moins les premières coordonnées d'emplacement par rapport aux troisièmes coordonnées d'emplacement afin de trouver les coordonnées d'emplacement relatives.

6. Dispositif selon la revendication 1, dans lequel le processeur de positionnement (38) est configuré pour comparer les premières coordonnées d'emplacement aux troisièmes coordonnées d'emplacement de manière à déterminer si l'écart est dû au déplacement de la sonde de référence (48) par rapport à l'emplacement de référence ou dû à un mouvement du corps du sujet (24).

7. Dispositif selon la revendication 1, dans lequel le processeur de positionnement (38) est configuré pour calculer un vecteur de correction en réponse au déplacement, et pour appliquer le vecteur de correction pour trouver les coordonnées d'emplacement relatives sur la base des premières et deuxièmes coordonnées d'emplacement.

8. Dispositif selon la revendication 7, dans lequel le processeur de positionnement (38) est configuré pour collecter et traiter d'autres coordonnées d'emplacement de la sonde de référence interne (48), après la détection de l'écart, de manière à définir une nouvelle plage des coordonnées d'emplacement correspondant à l'emplacement de référence, et pour calculer le vecteur de correction en comparant la nouvelle plage à la plage qui a été définie en collectant et en traitant les premières coordonnées d'emplacement.

9. Dispositif selon la revendication 1, dans lequel la sonde de référence interne (48) comprend une électrode, et dans lequel le processeur de positionnement (38) est couplé pour recevoir un signal d'électrogramme local de l'électrode à l'emplacement de référence dans le coeur (22) et pour détecter l'écart en détectant une variation du signal d'électrogramme local.
